Europäisches Patentamt

European Patent Office

Office européen des brevets

(11) Publication number: **0 425 828 A2**

# EUROPEAN PATENT APPLICATION

(21) Application number: 90118901.9

(51) Int. Cl.⁵: **A61K 37/02, A61K 37/24**

(22) Date of filing: 03.10.90

The applicant has subsequently filed a sequence listing and declared, that it includes no new matter.

The title of the invention has been amended (Guidelines for Examination in the EPO, A-III, 7.3).

(30) Priority: 04.10.89 JP 257729/89

(43) Date of publication of application:
08.05.91 Bulletin 91/19

(84) Designated Contracting States:
BE CH DE FR GB IT LI NL SE

(71) Applicant: **MITSUI TOATSU CHEMICALS, Inc.**
**2-5 Kasumigaseki 3-chome**
**Chiyoda-Ku Tokyo 100(JP)**

(72) Inventor: **Awaya, Akira**
**Dainiapato, 1541, Yabecho, Totsuka-ku**
**Yokohama-shi, Kanagawa-ken, 244(JP)**
Inventor: **Kobayashi, Hisashi**
**640-46, Miyanodaiapato, 2141, Togo**
**Mobara-shi, Chiba-ken, 297(JP)**
Inventor: **Abe, Hayao**
**640-16, Miyanodaiapato, 2141, Togo**
**Mobara-shi, Chiba-ken, 297(JP)**

(74) Representative: **Vossius & Partner**
**Siebertstrasse 4 P.O. Box 86 07 67**
**W-8000 München 86(DE)**

(54) **Facteur thymique sérique for prevention and remedy of disorders of decrease in the number of blood corpuscles.**

(57) A medicament for prevention and remedy of disorders of decrease in the number of blood corpuscles such as leukocytes, reticuloctyes and thrombocytes (blood platelets) and the use of this medicament are disclosed. The medicament contains as an effective ingredient thereof a nonapeptide having the following amino acid sequence:
pGlu-Ala-Lys-Ser-Gln-Gly-Gly-Ser-Asn
or an ester or an amide at the carboxyl group of the C-terminus of the asparagine or a pharmacologically acceptable salt thereof or a derivative thereof combined with a metal. This medicament is utterly non-toxic to human and animals and effective for prevention and remedy of disorders of decrease in the number of blood corpuscles such as leucocytes caused by administration of chemotherapeutics agents such as anti-cancer agents or accompanied by cancer or viral diseases.

EP 0 425 828 A2

# MEDICAMENT FOR PREVENTION AND REMEDY OF DISORDERS OF DECREASE IN THE NUMBER OF BLOOD CORPUSCLES

The present invention relates to a new medicament for human or animals which are useful for defense against and remedy of side-effects caused by administration of various chemotherapeutic agents. More particularly, the present invention relates to a medicament for recovery of hematopoietic dysfunction or for prevention and remedy of disorders of decrease in the number of blood corpuscles such as leucocytes, reticulocytes and thrombocytes caused by chemotherapeutic agents, in particular, anti-cancer agents. Further, the present invention relates to a medicament for prevention and remedy of disorders of decrease in the number of blood corpuscles such as leucocytes, reticulocytes and thrombocytes caused by serious sickness itself such as cancer or viral diseases caused, for example, by human immunodeficiency virus.

In chemotherapy of various diseases, such side-effects as hematotoxicity and hematopoietic dysfunction are deemed serious. In general, leucocytopenia, granulocytopenia and the like leucocyte disorder, anemia, hematocytopenia and the like hematocyte disorder, and thrombocytopenia are often caused by administration of various chemotherapeutic agents, especially anti-cancer agents, immuno-suppressive agents, antibiotics, and anti viral agents. Accordingly, development of a medicament is desired which recovers human or animals rapidly from such side-effects or which prevents or treats such side-effects. Up to the present, however, the number of such medicaments developed is rather small. Although a few medicaments such as cepharanthine, inosine, etc. are clinically applied, their pharmacological effects cannot be said to be sufficient.

Development of a medicament for prevention and remedy of various hematological disorders caused as side-effects by administration of a chemotherapeutic agent enables desirable increase in the quantity of administration of the chemotherapeutic agent to enhance the inherent therapeutic effect of human or animals on cancer or various diseases.

In the above mentioned circumstances, there is a great demand for developing a new medicament for prevention and remedy of various hematological disorders without any toxicity to human and animals.

The technical problem underlying the present invention is to provide pharmaceutical preparations for the treatment of various hematological disorders, particularly those diseases which accompany the administration of a chemotherapeutic agent or which accompany cancer or viral diseases. Specifically, these hematological disorders include disorders which result in a decrease in the number of blood corpuscles, such as leucocytes, reticulocytes and thrombocytes.

The solution to this technical problem is achieved by providing the pharmaceutical preparations of the present invention.

Accordingly, it is an object of the present invention to provide a medicament for prevention and remedy of disorders of decrease in the number of blood corpuscles caused by administration of a chemotherapeutic agent without any toxicity to human or animals.

It is another object of the present invention to provide a medicament for remedy of various hematological disorders accompanied with cancer or viral diseases without any toxicity to human or animals.

It is still another object of the present invention to use the medicament for prevention and remedy of disorders of decrease in the number of blood corpuscles caused by administration of a chemotherapeutic agent or accompanied with cancer or serious viral diseases.

Other and further objects, features and merits of the present invention will become apparent more fully from the following description.

With a view to developing a medicament which is different from chemotherapeutic agents and is capable of enhancing the defending ability inherently existing in living body, the present inventors have made extensive research on various peptides of natural origin higher in safety to determine a substance useful as a medicament for prevention and remedy of hematopoietic dysfunction and disorders of decrease in the number of blood corpuscles. As a result of the research, a nonapeptide known as the factor of thymic serum (Facteur thymique serique, referred to hereinafter simply as FTS) and derivatives or salts thereof, has been determined to be effective in preventing or treating disorders of decrease in the number of blood corpuscles such as leucocytes in mice caused by administration of a chemotherapeutic agent such as an anti-cancer agent or anti viral agent. It has also been determined that anemia and the like hematological disorder or symptoms of decrease in the number of blood corpuscles, such as leucocytopenia, accompanied by cancer or human immunodeficiency diseases can be prevented or treated effectively with FTS or derivatives or salts thereof. The present invention has been accomplished on the basis of the above studies.

One of the present inventors previously determined that FTS was suitable as a therapeutic agent for multiple sclerosis, Guillain-Barre syndrome, inflammatory neuritis, polyneuritis and other various diseases accompanying immunodeficiency such as immunological demyelinating diseases, and provided such therapeutic agent (Japanese Laid-open Patent Appln. No. Sho. 58-52225). However, the fact that FTS is highly effective for prevention and remedy of disorders of decrease in the number of blood corpuscles such as leucocytes was utterly unexpected from the prior art and for the first time determined by the present inventors.

In accordance with the present invention, there is provided a medicament for prevention and remedy of disorders of decrease in the number of blood corpuscles, characterized by containing as an effective ingredient thereof a nonapeptide having the following amino acid sequence:

pGlu-Ala-Lys-Ser-Gln-Gly-Gly-Ser-Asn or an ester or an amide at the carboxyl group of the C-terminus of the asparagine or a pharmacologically acceptable salt thereof or a derivative thereof combined with a metal.

In accordance with the present invention, there is also provided the use of the above nonapeptide for prevention and remedy of disorders of decrease in the number of blood corpuscles caused by administration of a chemotherapeutic agent or accompanied with cancer or viral diseases.

In the formula showing the above sequence, the symbols Glu, Ala, etc. stands for residues of amino acids. More precisely, pGlu stand for L-pyroglutamic acid, Ala for L-alanine, Lys for L-lysine, Ser for L-serin, Gln for L-glutamine, Gly for glycine and Asn for L-asparagine. As the terminal asparagine has a free carboxy group not participating in the polypeptide linkage, it may be converted at the carboxyl group into an ester or an amide thereof.

The nonapeptide used in the present invention is a known substance and can be prepared without difficulty according to a liquid phase or a solid phase peptide synthetic method conventionally employed for the synthesis of usual peptides (Concerning these methods, please refer to Japanese Laid-open Patent Appln. No. Sho. 54-16425 and U.S.P. 4,301,065). Alternatively, the nonapeptide can be prepared also by a genotechnological or cell technological procedure.

The esters at the carboxyl group of the C-terminus of the asparagine in the nonapeptide used in the present invention are those of the pharmacologically acceptable carboxylic acid and examples of such esters include methyl ester, ethyl ester, propyl ester, isopropyl ester, n-butyl ester, isobutyl ester, tert-butyl ester, n-pentyl ester, isopentyl ester, neopentyl ester, tert-pentyl ester, n-hexyl ester, sec-hexyl ester, heptyl ester, octyl ester, sec-octyl ester, tert-octyl ester, nonyl ester, decyl ester, undecyl ester, dodecyl ester, tridecyl ester, tetradecyl ester, hexadecyl ester, octadecyl ester, nonadecyl ester, eicocyl ester, cyclopentyl ester, cyclohexyl ester, cycloheptyl ester, cyclooctyl ester, allyl ester, isopropenyl ester, benzyl ester, o-, m- or p-chlorobenzyl ester, o-, m- or p-fluorobenzyl ester, o-, m- or p-bromobenzyl ester, o-, m- or p-iodobenzyl ester, o-, m- or p-methylbenzyl ester, o-, m- or p-ethylbenzyl ester, o-, m- or p-isopropylbenzyl ester, cinnamyl ester, aminoethyl ester, o-, m- or p-aminobenzyl ester, o-, m- or p-nitrobenzyl ester, o-, m- or p-methoxybenzyl ester, o-, m- or p-ethoxybenzyl ester, o-, m- or p-aminophenetyl ester, $\alpha$-furfuryl ester, $\alpha$-thienylmethyl ester, $\alpha$-pyridylmethyl ester, $\alpha$-pyridylethyl ester, piperidinomethyl ester, $\alpha$-piperidylmethyl ester, morpholinomethyl ester, $\alpha$-morpholinylmethyl ester. The amides at the carboxyl group of the C-terminus of the asparagine in the nonapeptide used in the present invention are those of the pharmacologically acceptable carboxylic acid and examples of such amides include amide itself, methylamide, ethylamide, propylamide, isopropylamide, n-butylamide, isobutylamide, tert-butylamide, n-pentylamide, isopentylamide, neopentylamide, tert-pentylamide, n-hexylamide, sec-hexylamide, heptylamide, octylamide, sec-octylamide, tert-octylamide, nonylamide, decylamide, undecylamide, dodecylamide, tridecylamide, tetradecylamide, hexadecylamide, octadecylamide, nonadecylamide, eicosylamide, cyclopentylamide, cyclohexylamide, cycloheptylamide, cyclooctylamide, allylamide, isopropenylamide, benzylamide, o-, m- or p-chlorobenzylamide, o-, m- or p-fluorobenzylamide, o-, m- or p-bromobenzylamide, o-, m- or p-iodobenzylamide, o-, m- or p-methylbenzylamide, o-, m- or p-ethylbenzylamide, o-, m- or p-isopropylbenzylamide, cinnamylamide, aminoethylamide, o-, m- or p-aminobenzylamide, o-, m- or p-nitrobenzylamide, o-, m- or p-methoxybenzylamide, o-, m- or p-ethoxybenzylamide, o-, m- or p-aminophenethylamide, $\alpha$-furfurylamide, $\alpha$-thienylmethylamide, $\alpha$-pyridylmethylamide, $\alpha$-pyridylethylamide, piperidinomethylamide, $\alpha$-piperidylmethylamide, morpholinoethylamide, $\alpha$-morpholinylmethylamide, methoxycarbonyl-($\alpha$-mercapto-methyl)methylamide and ethoxycarbonyl-($\alpha$-mercapto-methyl)methylamide.

The above mentioned pharmacologically acceptable salts include acid-additions salts at the amino group of the nonapeptide and salts with bases at the carboxylic acid of the nonapeptide. The acid-addition salts include those with organic acids and inorganic acids. Illustrative of these salts are, for example, salts with carboxylic acids such as formic acid, acetic acid, propionic acid, trifluoroacetic acid, tartaric acid, fumaric acid, malic acid, maleic acid, oxalic acid and naphthoic acid and salts with sulfonic acids such as methanesulfonic acid, p-toluenesulfonic acid and naphthalenesulfonic acid as well as salts with inorganic acids such as hydrochloric acid, sulfuric acid, nitric acid and phosphoric acid.

3

The above mentioned salts with bases include salts with inorganic bases, such as alkali metal salts, alkaline earth metal salts and ammonium salts as well as salts with organic bases, i.e. salts with amines. Illustrative of these salts are lithium salt, sodium salt, potassium salt, calcium salt, ammonium salt, triethyl amine salt, ethanolamine salt, tris salt and dicyclohexylamine salt.

The nonapeptide may also be in the form combined with a metal. Illustrative of the metal to be combined are, for example, zinc, iron, cobalt, nickel, magnesium, chromium, gold, germanium, garium, potassium and calcium. These metals may present in monovalent, bivalent, trivalent or tetravalent form.

The medicament of this invention as an agent for prevention and remedy of decease in the number of leucocytes, reticulocytes and platelets can be prepared in a conventional pharmaceutical manner according to the type of the medicament. The effective ingredient selected from the nonapeptide, an ester, an amide, and a salt thereof is processed properly with a pharmacologically acceptable carrier, excipient or diluent to a suitable type of medicament. The medicament can be made in any of the various types suitable for the route of administration such as external application, oral administration, non-oral administration, etc.

The effect of the medicament of this invention as an agent for prevention and remedy of decrease in the number of leucocytes, reticulocytes and platelets and of hematopoietic dysfunction caused by administration of an anti-cancerous agent, etc. is confirmed by the experiments as described below.

Using a test mammal such as a mouse, rat, guinea pig, dog, monkey, baboon, etc., a substance capable of causing suppression of bone narrow function, leucocytopenia, hematocytopenia, reticulocytopenia, anemia, thrombocytopenia, etc. is administered in a given amount orally or non-orally to the test animal. Such substance include various anti-cancer agents, for example, cyclophosphamide (referred to hereinafter simply as CPA), mitomycine C (referred to hereinafter simply as MMC), 5-fluorouracil (referred to hereinafter simply as 5-FU), adriamycin (referred to hereinafter simply as ADM), daunomycin, aclacinomycin, neocarzinostatin, vincristine, vinblastine, vindesine sulfate, nitromine, triethylenethiophosphoramide (TESPA), carboquone (CQ), busulfan, improsulfan tosylate (864-T), nimustine hydrochloride (ACNU), melphalan, mitobronitol, ranimustine (MCNU), ifosfamido, methotrexate, 6-mercaptopurine, thioinosine, tegafur, carmofur, UFT, $5'$-DFUR, cytarabine (Ara-C), Cyclo-C, mitoxatrone, actinomycin D. chromomycin $A_3$, L-asparaginase, tamoxifen, cisplatin, estramustine, etoposide, interferon and lentinan. A blood inspection test is performed for the test animal after the lapse of a given period of time from the administration of the anti-cancer agent to check the number of leucocytes, hematocytes, reticulocytes, platelets and hemoglobin, a hematocrit value, a mean corpuscular volume, an amount of a mean corpuscular hemoglobin and a mean corpuscular hemoglobin concentration. Prior to or just after administration of the anti-cancer agent, a given amount of the nonapeptide is administered in various routes to the test animal, e.g. intraperitoneally, subcutaneously, intramuscularly, intravenously or orally and results of the experiments concerning the blood inspection and body weight are compared between the group to which the nonapeptide was administered and control group. According to a result of the experiment, it is observed that the degree of hematopoietic dysfunction of the animal in the control group is enhanced according to increase in the amount of an anti-cancer agent administered, thus showing decrease in the number of blood corpuscles and in various parameters of blood and suppression of increase in body weight for a certain period of time. On the other hand, it is observed that in the group of animals to which the nonapeptide was administered, decrease in the number of blood corpuscles is inhibited and recovery of the number of blood corpuscles to a normal range is also promoted, while suppression of increase in body weight is reduced. In addition, decrease in the number of corpuscles caused by administration of various chemotherapeutic agents such as bactericidal agents, anti viral agents or immunoinhibitory agents or of a substance known as causing decrease in the number of blood corpuscles is inhibited and recovery of the number of blood corpuscles to a normal range is promoted by administration of the nonapeptide of the present invention.

It is manifested from results of these experiments that the nonapeptide acts on hematogenic organs such as bone marrow or spleen to show inhibition to hematopoietic dysfunction and promotion of recovery action to such dysfunction, and as a result, the nonapeptide can be used as a valuable medicament for prevention and remedy of leucocytopenia, hematocytopenia and other hematopoietic dysfunction.

To check any toxicity of the effective ingredient of the medicament of this invention, 100 mg/kg of the effective ingredient was subcutaneously administered every day for consecutive 14 days to mice whereupon no abnormal symptom was found by external check. Further, 30 mg/kg of the effective ingredient was subcutaneously administered to rats for consecutive 21 days whereupon no abnormal symptom was found in external behavior observation and biochemical diagnosis of sera and in a result of pathological anatomy. Thus, the medicament of the present invention is a safe medicament of extremely low toxicity and can be administered for a long period of time.

As animals to which the medicament of this invention can be administered, there can be mentioned, for example, human, domestic animals such as cattle, horse, pig, sheep, goat, rabbit, dog and cat, mammals kept in zoo or the like such as lion, elephant, giraffe, bear, gorilla, monkey and chimpanzee, various test animals such as mouse, rat, guinea pig and the like, domestic fowls such as fowl, and pets such as birds, reptiles, amphibia and fishes. The dose of the medicament is usually 0.1 $\mu$g - 500 mg/day for 1 kg body weight of these animals. The medicament in these doses may be administered, for example, in 1-6 portions in a day. The dose may properly be increased or decreased according to ages, symptoms, etc. of the objects to be administered. No limitation exists in the route of administration of the medicament, but it may be administered by intravenous, intramuscular intracutaneous and subcutaneous injection. The medicament can be processed to an ointment which is applied to eyes, oral cavity, nasal cavity and skin. The medicament can be administered in the form of a suppository, a jelly, eye drops, nasal drops, preparations absorbable in nasal or oral cavity, an aerosol, a spray and oral preparations. In order to prevent the effective ingredient from rapid decomposition or inactivation in the living body, the effective ingredient may be processed with appropriate pharmaceutical ingredients, for example, alcoholic, oily or fatty physiologically harmless solid or liquid materials such as lecithin or a suspension liposome thereof to medical preparations in which the activity is maintained for a long period of time. The medicament of the present invention can be administered together with various other medicines, for example, a biological response modifier such as an immunostimulent and/or preparations possessing a recovery action against leucopenia, such as glutathione preparations, inosine preparations, adenine preparations and cepharanthine preparations, or alternatively may be incorporated with these preparations as a combination agent to enhance clinical effect.

The present invention will now be illustrated in more detail by way of examples and experimental examples.

Example 1 A vial preparation for injection

In a distilled water was dissolved 1 mg of ETS$\cdot$CH$_3$COOH$\cdot$2H$_2$O (prepared by Mitsui Pharmaceuticals, Inc.) and the solution was subjected to sterilizing filtration, charged into a vial and then subjected to lyophilization.

Example 2 An ampoule preparation for injection

In physiological saline was dissolved 5 mg of FTS$\cdot$CH$_3$COOH$\cdot$2H$_2$O (prepared by Mitsui Pharmaceuticals, Inc.) and the solution was subjected to sterilizing filtration and charged into an ampoule.

Example 3 An injection preparation for subcutaneous injection

In a 2% carboxymethylcellulose PBS (physiological saline buffered with a phosphate) was suspended 2 mg per unit dose of FTS$\cdot$CH$_3$COOH$\cdot$2H$_2$O (prepared by Mitsui Pharmaceuticals, Inc.). The suspension was mixed with Lipomal comprising soybean phosphatide (prepared by Huhtamaki Oy/Leiras Pharmaceuticals Co.) or Intralipid (prepared by Cutter Laboratories) as an oil-in-water type emulsion for intravenous injection. In case of using Lipomal, the PBS solution dissolving FTS was mixed with an equiamount of Lipomal. In case of using Intralipid, 2.5 ml of the PBS solution dissolving FTS was mixed with 0.1 ml of Tween 80 (prepared by Sigma Chemicals Inc.) and 4.6 ml of Intralipid.

Example 4 A liposome preparation

There are 3 kinds of liposome preparations which are different in electric charge from one another. The liposome preparations are classified by their structures into 4 kinds.

There are 3 kinds of ionic charge: neutral, cationic and anionic. In view of the structure, there are known 4 kinds; a multilaminar liposome (MLV, multilamellar vesicle), a small unilaminar liposome (SUV, small unilamellar vesicle), a large unilamilar liposome (LUV, large unilamellar vesicle) and one having a structure similar to LUV but having several lamellae (REV, reverse-phase evaporation vesicle).

(1) A neutral ionic charge liposome enclosing FTS:

5

A phospholipid such as phosphatidylcholine or sphingomyelin and a solution of cholesterol in chloroform were mixed in a mole ratio of 2:1, 4:1 or 1:1 and the solvent was once removed from the mixture by distillation under reduced pressure. A solution of 1/100 - 1/1000 equivalent of FTS in PBS (physiological saline buffered with a phosphate) was added to the mixture and the whole was well mixed by the aid of a Vortex mixer whereby MLV was obtained.

This was then subjected to an ultrasonic treatment above a phase transition temperature (Tc) of the phospholipid whereby SUV was obtained.

An aqueous solution of calcium chloride was added to the resultant SUV and the mixture was incubated for 1 hour at $37^\circ$C to effect hybridization. EDTA was then added and the mixture was incubated for 30 minutes at $37^\circ$C to eliminate $Ca^{++}$ whereby LUV was obtained.

The method for preparing REV is as follows: After removing the solvent from a chloroform solution of the lipid by distillation under reduced pressure, a proper amount of diethyl ether is added to dissolve the lipid satisfactorily. A PBS solution of FTS is added to the solution and the mixture is subjected to an ultrasonic treatment to obtain a homogenous uniphase solution. After concentrating the resultant solution under reduced pressure at room temperature, the PBS solution is added to the residue and the mixture was mixed well by the aid of a Vortex mixer to obtain REV.

(2) A cationic liposome enclosing FTS:

Except that the constituents of the lipid are different, the method for preparing the liposome is same as that in case of the above mentioned neutral ionic charge liposome .

A phospholipid such as phosphatidylcholine or sphingomyelin, cholesterol, and a cationic higher aliphatic amine such as stearylamine were mixed together in a molar ratio of 7:2:1 or 4:1:1 to form a lipid ingredient, and FTS was enclosed in a similar manner.

(3) A anionic liposome enclosing FTS:

A phospholipid such as phosphatidylchlorine or sphingomyelin, cholesterol, and a anionic higher aliphatic ester such as dicetyl phosphate or sulfatide were mixed together in a molar ratio of 7:1:1 or 4:1:1 to form a lipid ingredient, and FTS was enclosed in a similar manner.

Example 5 An ointment

In purified water was dissolved 2 mg of $FTS \cdot CH_3COOH \cdot 2H_2O$ (prepared by Mitsui Pharmaceuticals, Inc.). Next, 25 g of white vaseline, 20 g of stearyl alcohol, 4 g of HCO-60 and 1 g of glycerol monostearate were weighed and mixed. A previously prepared aqueous solution (containing FTS) of 12 g of propylene glycol, 0.1 g of methyl p-hydroxybenzoate and 0.1 g of propyl p-hydroxybenzoate was added to the mixture and the whole was thoroughly blended to form an emulsion which was then mixed until it was cooled and solidified.

Example 6 A suppository

In a hard fat previously warmed was dispersed 10 mg of $FTS \cdot CH_3COOH \cdot 2H_2O$ (prepared by Mitsui Pharmaceuticals, Inc.). The total amount was adjusted to 2 g.

Example 7 A capsule for nasal use

In 29.95 mg of miglyol 812 neutral oil (Dynamite Nobel Co.) was dissolved under a sterilizing condition 0.05 mg of FTS. This solution was charged into a conventional unit-dose capsule which was treated with an applying device just before the use.

Example 8 Nasal drops

6

In distilled water were dissolved at room temperature sodium dihydrogen phosphate, disodium hydrogen phosphate, sodium chloride and EDTA-disodium salt in amounts as shown below. FTS was dissolved in this solution which was then filtered through a membrane filter.

| | |
|---|---|
| FTS | 0.10 mg |
| Sodium monohydrogen phosphate $2H_2O$ | 0.30 mg |
| Sodium dihydrogen phosphate $12H_2O$ | 10.10 mg |
| Benzalkonium chloride | 0.10 mg |
| Ethylenediamine tetraacetic acid disodium salt (EDTA) | 0.50 mg |
| Sodium chloride | 4.50 mg |
| Distilled water | 987.60 mg |
| pH value | 5.0±0.3 |

Example 9 A nasal spray preparation

In hydroxypropylcellulose or hydroxypropylmethyl-cellulose was suspended $FTS \cdot CH_3COOH \cdot 2H_2O$ (prepared by Mitsui Pharmaceuticals, Inc.). The suspension was processed to a spray preparation by the aid of a spray-preparing machine.

Given below are examples for pharmacological experiments and toxicity experiments concerning the medicament of this invention.

Experimental Example 1

Effect on mice to which mitomycin (MMC) was orally administered

Four groups each consisting of 3 female ICR mice (Japan CLEA) of 7 weeks old were treated as follows: For the first group, 0.1 ml of physiological saline was intraperitoneally administered to the mice for consecutive 4 days. For the second group, $FTS \cdot CH_3COOH \cdot 2H_2O$ used in Example 1 (referred to hereinafter simply as FTS substance) was intraperitoneally administered in an amount of 100 $\mu$g once a day to the mice for consecutive 4 days. For the third group, MMC in an amount of 5 mg/kg was orally administered to the mice only once at the start of the examination. For the fourth group, FTS substance in an amount of 100 $\mu$g was intraperitoneally administered to the mice once a day for consecutive 4 days from two days before the administration of MMC, and on the third day from the administration of FTS substance MMC was once orally administered in an amount of 5 mg/kg. Just before the administration of medicaments, blood was collected daily from oculus vein and, after dyeing with the Turk solution, the number of leucocytes was counted. A result of the experiment is shown in Table 1. As is evident from Table 1, FTS substance was found to prevent or suppress the temporary decrease in the numbr of leucocytes in peripheral blood on the day succeeding the administration of MMC, and so evaluation of FTS substance as an agent for prevention and remedy of hematopoietic dysfunction caused by the administration of the anti-cancer agent was made clear.

7

Table 1

| Effect on mice to which MMC was orally administered | | | | | |
|---|---|---|---|---|---|
| | The number of leucocytes in peripheral blood of each mouse (cells/mm$^3$) [mean value ± S.D.] | | | | |
| | Day of the examination | | | | |
| Substance administered | -2 | -1 | 0 | 1 | 2 |
| Physiological saline | 6950 | 6825 | 7200 | 10000 | 11000 |
| | 9850 | 6500 | 8800 | 9025 | 9300 |
| | 7300 | 7725 | 6700 | 8050 | 8375 |
| | [8033±1583] | [7017±635] | [7567±1097] | [9025±975] | [9558±1331] |
| FTS substance | 7100 | 9250 | 7725 | 9025 | 10525 |
| | 7400 | 8025 | 7325 | 7725 | 9875 |
| | 7575 | 10300 | 7525 | 6100 | 9450 |
| | [7358±240] | [9192±1139] | [7525±200] | [7617±1466] | [9950±541] |
| MMC | 7250 | 10800 | 9000 | 4050 | 7000 |
| | 8500 | 8250 | 8025 | 3000 | 10275 |
| | 7625 | 7300 | 7275 | 4675 | 8050 |
| | [7792±641] | [8783±1810] | [8200±728] | [3908±846] | [8442±1672] |
| MMC + FTS substance | 7725 | 5900 | 7250 | 5950 | 8600 |
| | 7375 | 6800 | 9700 | 6050 | 8100 |
| | 7475 | 11175 | 8875 | 9050 | 9950 |
| | [7525±180] | [7958±2822] | [8608±1247] | [7017±1762] | [8883±957] |

Experimental Example 2

Effect on mice to which 5-fluorouracil (5-FU) was orally administered

An experiment was carried out in the same manner as described in Experimental Example 1 except that the group of the mice to which 5-FU was orally administered once in an amount of 50 mg/kg was used as the fifth group in place of the third group in Experimental Example 1. In addition, a group of the mice to which the FTS substance was intraperitoneally administered once a day in an amount of 100 $\mu$g for consecutive 4 days from the two days before the administration of 5-FU and on the third day from the administration of FTS substance, i.e. on the zero day of the experiment, 5-FU was once orally administered in an amount of 50 mg/kg was used as the sixth group. The examination was then performed in the same manner as in Experimental Example 1. A result obtained is shown in Table 2. It is evident from Table 2 that FTS substance was found to prevent or suppress the decrease in the number of leucocytes caused by the administration of 5-FU.

Table 2

| Effect on mice to which 5-FU was orally administered | | | | | |
|---|---|---|---|---|---|
| | The number of leucocytes in peripheral blood of each mouse (cells/mm$^3$) [mean value ± S.D.] | | | | |
| | Days of the examination | | | | |
| Substance administered | -2 | -1 | 0 | 1 | 2 |
| Physiological saline | 6950 | 6825 | 7200 | 10000 | 11000 |
| | 9850 | 6500 | 8800 | 9025 | 9300 |
| | 7300 | 7725 | 6700 | 8050 | 8375 |
| | [8033±1583] | [7017±635] | [7567±1097] | [9025±975] | [9558±1331] |
| FTS substance | 7100 | 9250 | 7725 | 9025 | 10525 |
| | 7400 | 8025 | 7325 | 7725 | 9875 |
| | 7575 | 10300 | 7525 | 6100 | 9450 |
| | [7358±240] | [9192±1139] | [7525±200] | [7617±1466] | [9950±541] |
| 5-FU | 7475 | 8825 | 7275 | 5500 | 10000 |
| | 7200 | 10800 | 9050 | - | - |
| | 7550 | 10700 | 9275 | 5575 | 10550 |
| | [7408±184] | [10108±1113] | [8533±1096] | [5538±53] | [10275±389] |
| 5-FU + FTS substance | 6975 | 10650 | 9225 | 7100 | 10625 |
| | 8025 | 8800 | 8775 | 8025 | 9150 |
| | 7575 | 7200 | 9200 | 5825 | 7900 |
| | [7525±527] | [8883±1727] | [9067±253] | [6983±1105] | [9225±1364] |

Experimental Example 3

Effect on mice to which MMC was intraperitoneally administered

Four groups each consisting of 6 female ICR/JCL mice of 5 weeks old were treated as follows: For the first group, 0.1 ml of physiological saline was intraperitoneally administered to the mice for consecutive 12 days. For the second group, FTS substance was subcutaneously administered once a day in an amount of 100 μg for consecutive 12 days. For the third group, MMC was intraperitoneally administered once a day in an amount of 20 μg/0.1 ml for 10 days from the zero day to the 9th day of the experiment. For the fourth group, FTS substance was subcutaneously administered to the mice once a day in an amount of 100 μg for consecutive 12 days from 2 days before the administration of MMC, and for 10 days from the third day after the administration of FTS substance MMC was intraperitoneally administered once a day in an amount of 20 μg/0.1 ml. After the administration of the anti-cancer agent, blood was collected on alternate days from oculus vein and the numbr of leucocytes in the blood was counted. A result of the experiment is shown in Table 3.

No significant difference was found in the number of leucocytes in peripheral blood between the group of the mice to which FTS substance alone was administered and the group of the mice to which physiological saline was administered. The difference was within the normal range. In the group of the mice to which MMC alone was administered, the number of leucocytes began to decrease from 3 days after the initial administration and reached about 40% of the maximum normal range. This decreasing tendency was observed for more than one week even after completion of the administration of MMC. On the other hand, it was observed that the number of leucocytes in the group of the mice to which MMC was jointly administered with FTS substance was maintained at about 60-80% of the group of the mice to which physiological saline was administered and that the recovery of the number to the normal range was promoted after completion of the administration of the anti-cancer agent.

Table 3

EP 0 425 828 A2

| Effect on mice to which MMC was intraperitoneally adminstered | | | | | | | | |
|---|---|---|---|---|---|---|---|---|
| The number of leucocytes in peripheral blood of each mouse (cells/mm$^3$) [mean value ± S.D.] | | | | | | | | |
| Day of the examination | | | | | | | | |
| Substance administered | 0 | 1 | 3 | 5 | 7 | 10 | 12 | 14 | 17 |
| Physiological saline | 7454±2726 | 10088±1964 | 8504±1538 | 9188±1384 | 9815±1161 | 8050±943 | 8055±1833 | 8119±2159 | 8044±862 |
| FTS substance | 6058±943 | 9613±2888 | 9210±2726 | 8965±1575 | 9500±1288 | 8581±1636 | 8144±961 | 7419± 548 | 8075±661 |
| MMC | 6958±2288 | 9420±2839 | 4654±2564 (**) | 6408±2272 (*) | 5365±1732 (***) | 3725±907 (***) | 3533± 739 (**) | 4092±1338 (*) | 4668±1617 (**) |
| MMC + FTS substance | 5975±1485 (*) | 6175±1151 (*** #) | 5610±1457 (***) | 5040±1196 (***) | 6775±1034 (***) | 4840±674 (***) | 6445±1101 (* ##) | 6515±1114 (* #) | 6115±685 (***) |

Inspection of significant difference:

for the group to which physiological saline was administered; * P<0.05, ** P<0.01, *** P<0.001

for the group to which MMC alone was administered; # P<0.05, ## P<0.01

Experimental Example 4

Effect on mice to which 5-FU was intraperitoneally administered

An experiment was carried out in the same manner as described in Experimental Example 3 except that a group of the mice to which 5-FU was intraperitoneally administered once a day in an amount of 60 μg/0.1 ml for 10 days from the zero day to the 9th day of the experiment was used as the fifth group in place of the third group in Experiment Example 3. In addition, a group of the mice to which FTS substance was subcutaneously administered once a day in an amount of 100 μg for consecutive 12 days from two days before the administration of 5-FU, and for 10 days from the third day after the administration of FTS substance 5-FU was intraperitoneally administered once a day in an amount of 60 μg/0.1 ml to the mice was used as the sixth group. The examination was then performed in the same manner as in Experimental Example 3. A result obtained is shown in Table 4. In the group of the mice to which 5-FU alone was administered, the number of leucocytes began to decrease from 3 days after the initial administration and reached about 37% of the maximum normal range. This decreasing tendency was observed for more than one week even after completion of the administration of 5-FU. On the other hand, it was observed that the number of leucocytes in the group of the mice to which 5-FU was jointly administered with FTS substance was maintained at about 60-90% of the group of the mice to which physiological saline was administered and that the recovery of the number to the normal range was promoted after completion of the administration of the anti-cancer agent.

Table 4

Effect on mice to which 5-FU was intraperitoneally administered

| Substance administered | The number of leucocytes in peripheral blood of each mouse (cells/mm$^3$) [mean value ± S.D.] | | | | | | | | |
|---|---|---|---|---|---|---|---|---|---|
| | Day of the examination | | | | | | | | |
| | 0 | 1 | 3 | 5 | 7 | 10 | 12 | 14 | 17 |
| Physiological saline | 7454±2726 | 10088±1964 | 8504±1538 | 9188±1384 | 9815±1161 | 8050±943 | 8055±1833 | 8119±2159 | 8044±862 |
| FTS substance | 6058±943 | 9613±2888 | 9210±2726 | 8965±1575 | 9500±1288 | 8581±1636 | 8144±961 | 7419±548 | 8075±661 |
| 5-FU | 5367±1198 (*) | 8767±2070 | 5404±2671 (**) | 5335±1451 (***) | 3705±746 (***) | 3567±317 (***) | 3433±138 (***) | 3525±400 (**) | 3708±912 (***) |
| 5-FU + FTS substance | 5508±868 (**) | 7896±1450 (***) | 5933±2141 (***) | 7675±2646 (*) | 5990±1228 (*** ※※) | 6206±1007 (*** ※※) | 5481±2479 (**) | 5175±1134 (** ※) | 7394±1304 (※※) |

Inspection of significant difference:
for the group to which physiological saline was administered; * $P<0.05$, ** $P<0.01$, *** $P<0.001$
for the group to which 5-FU alone was administered; ※ $P<0.05$, ※※ $P<0.01$

Effect on mice to which cyclophosphamide (CPA) was intraperitoneally administered

Several groups each consisting of 10 male C3H/HeN mice of 8 weeks old were prepared. For the first group, 0.2 ml of physiological saline was subcutaneously administered for consecutive 12 days from 2 days before the examination to the 9th day of the examination. For the second group, FTS substance was subcutaneously administered once a day in an amount of 100 $\mu$g/0.2 ml for consecutive 12 days. For the third group, CPA was intraperitoneally administered in an amount of 400 mg/kg once on the zero day of the examination. For the fourth group, an administration schedule combining the second group with the third group was applied to the mice. For the fifth group, CPA was intraperitoneally administered in an amount 200 mg/kg once on the zero day of the examination. For the sixth group, an administration schedule combining the second group with the fifth group was applied to the mice. For the seventh group, CPA was intraperitoneally administered in an amount of 100 mg/kg once on the zero day of the examination. For the eighth group, an administration schedule combining the second group with the seventh group to the mice. During the examination, blood was collected by cutting axillary artery on the days of examination (0, 3rd, 5th, 7th, 10th and 14th days) and subjected to a blood inspection to measure the number of leucocytes (WBC), the number of erythrocytes (RBC), the number of reticulocytes (RET), the number of platelets (PLT), the number of hemoglobin (HGB), hematocrit value (HCT), a mean corpuscular volume, a mean corpuscular hemoglobin and a mean corpuscular hemoglobin concentration.

In the group of mice to which CPA alone was administered in an amount of 400 mg/kg, 200 mg/kg or 100 mg/kg, WBC was considerably decreased on the 3rd, 5th and 7th day of the examination, with the 5th day being the minimum. In the group of the mice to which FTS substance was jointly administered, however, decrease in WBC was suppressed. For example, a mean value ± S.D. of WBC on the 3rd day of the examination was 45.2±5.0 ($\times$ $10^2$/mm$^3$) in the group of the mice to which physiological saline was administered, while the value was 48.0±4.7 in the group of the mice to which FTS substance alone was administered, 11.6±2.1 in the group of the mice to which 400 mg/kg of CPA alone was administered or 17.0±2.0 in the group of the mice to which CPA was jointly administered with FTS substance. Further, a mean value ± S.D. of WBC on the 5th day of the examination was 41.8±6.5 in the group of the mice to which physiological saline was administered, while the value was 38.0±6.1 in the group of the mice to which FTS substance alone was administered, 4.2±1.0 in the group of the mice to which 400 mg/kg of CPA alone was administered, or 6.4±0.3 in the group of the mice to which CPA was administered jointly with FTS substance. In the groups of the mice to which FTS substance was jointly administered, decrease in WBC was reduced and recovery of WBC was promoted on 7th and 10th days of the examination.

In the groups of the mice to which CPA was administered, RET was decreased with the 5th day being the minimum. However, RET of the groups of the mice to which CPA was administered jointly with FTS substance was recovered to the normal range on the 7th day in case of 200 mg/kg or 100 mg/kg of CPA being administered. For example, RET in the group of mice to which physiological saline was administered was 52.6±7.3 (0/00) on the 7th day, while RET was 68.8±12.0 in the group of the mice to which FTS substance alone was administered, 27.2±5.0 in the group of the mice to which 400mg/kg of CPA alone was administered, or 40.8±7.2 in the group of the mice to which CPA was administered jointly with FTS substance. Decrease in RET was suppressed in the groups of the mice to which FTS substance was jointly administered, and recovery of RET was promoted.

In the groups of the mice to which 400 mg/kg and 200 mg/kg of CPA were administered, PLT was decreased with the 7th day being the minimum. However, the value was recovered to the normal range on the 10th day. For example, PLT on the 5th day was 933±26.0 ($\times$ $10^3$/mm$^3$) in the group of the mice to which physiological saline was administered, while PLT was 804±162 in group of the mice to which FTS substance alone was administered, 357±82 in the group of the mice to which 400 mg/kg of CPA alone was administered, or 580±49 in the group of the mice to which CPA was administered jointly with FTS substance. Further, PLT on the 7th day was 724±60 in the group of the mice to which physiological saline was administered, while PLT was 707±172 in the group of the mice to which FTS substance alone was administered, 214±73 in the group of the mice to which 400 mg/kg of CPA alone was administered, or 540±87 in the group of the mice to which CPA was administered jointly with FTS substance. Decrease in PLT was suppressed in the groups of the mice to which FTS substance was jointly administered, and recovery of PLT was promoted.

In the groups of the mice to which FTS substance was administered jointly, decrease in other blood parameters of the blood inspection, i.e. RBC, HGB, HCT, etc. was suppressed, and recovery of these blood parameters was promoted. It is also manifested that decrease in body weight as seen in the group of the mice to which CPA alone was administered was prevented in the groups of the mice to which FTS substance was jointly administered.

Experimental Example 6 Toxicity test

No toxicity was observed when 50 mg/kg and 100mg/kg of the effective ingredient were subcutaneously administered for consecutive 14 days to a group of five ddy strain male mice of 5 weeks old.

Experimental Example 7 Toxicity test

No toxicity was observed when 30 mg/kg of the effective ingredient was subcutaneously administered for consecutive 21 days to a group of ten Wistar rats of 5 weeks old.

In the medical field for preventing blood deficiencies such as leucocytopenia and hematopoietic dysfunction where no effective medicament exists except a conventional agent for recovery of leucocytopenia, the medicament of this invention is indeed epoch-making in bringing about an excellent therapeutic effect on leucocytopenia and hematopoietic dysfunction by activating the systems of immunity and living body defense. The nonapeptide (FTS) used in the medicament of this invention is a peptide originating from animals and is a natural substance. Accordingly, it is quite non-toxic in living body and has no problem of anti genicity of anaphylactic shock, unlike the case of FTS analogs having a similar amino acid sequence.

Thus, the medicament concerned with the present invention can be used as safe and useful medical preparations and veterinary medical preparations.

It is understood that the preceeding representative examples may be varied within the scope of the present specification, both as to the ingredients and conditions, by those skilled in the art to achieve essentially the same results.

As many apparently widely different embodiments of the present invention may be made without departing from the spirit and scope thereof, it is to be construed that the present invention is not limited to the specific embodiments thereof as defined in the appended claims.

**Claims**

1. The use of a nonapeptide having the following amino acid sequence:
pGlu-Ala-Lys-Ser-Gln-Gly-Gly-Ser-Asn
or an ester or an amide at the carboxyl group of the C-terminus of the asparagine or a pharmacologically acceptable salt thereof or a derivative thereof combined with a metal for producing a medicament for prevention and remedy of hematological disorders.

2. The use according to claim 1 or wherein said hematological disorders are disorders which result in the decrease in the number of blood corpuscles.

3. The use according to claim 2 wherein said decrease in the number of blood corpuscles is caused by the administration of a chemotherapeutic agent.

4. The use according to claim 2 wherein said decrease in the number of blood corpuscles accompanies cancer or viral diseases.

5. The use according to any one of claims 2 to 4 wherein said blood corpuscles are leucocytes, reticulocytes or platelets.